# EUROPEAN PATENT APPLICATION

(11) **EP 3 721 899 A1**
(43) Date of publication of application: **14.10.2020**
(21) Application number: 19167917.4
(22) Date of filing: 08.04.2019
(51) Int. Cl.: A61K 39/00, A61P 35/00, C07K 16/00

(54) **COMBINATION THERAPIES COMPRISING DENDRITIC CELLS-BASED VACCINE AND IMMUNE CHECKPOINT INHIBITOR**

(71) Applicant: China Medical University, Taichung (TW)
(72) Inventor: JENG, Long-Bin, Taichung (TW); SHYU, Woei-Cherng, Taichung (TW); TENG, Chiao-Fang, Taichung (TW); TSAI, Chang-Hai, Taichung (TW)
(74) Representative: J A Kemp LLP

(57) **Abstract**

The present disclosure provides a method for treating HCC comprising co-administering to the patient, dendritic cells-based vaccine in combination with an immune checkpoint inhibitor. The treatment with DC vaccine in combination with immune checkpoint inhibitor has significantly improved overall survival in subjects.

## Description

### Field of the Invention

The present invention relates to the field of medicine. Particularly, the present invention pertains to treatment of a cancer (preferably, hepatocellular carcinoma (HCC)) using dendritic cells-based vaccine and an immune checkpoint inhibitor.

### Background of the Invention

Hepatocellular carcinoma (HCC) is the most common of the hepatobiliary (liver, gall bladder and bile duct) cancers. The pathogenesis of HCC has been associated with chronic hepatitis B virus (HBV) and hepatitis C virus (HCV) infections, as well as cirrhosis-inducing conditions of liver. Risk factors for HCC include infection with hepatitis B virus (HBV) or hepatitis C virus (HCV), alcoholic cirrhosis, and other liver conditions, such as hemochromatosis or late stage primary biliary cirrhosis (PBC). Multiple therapeutic approaches have been developed for the treatment of HCC, including surgical resection, liver transplantation, and many nonsurgical therapeutic options, such as radiofrequency ablation, transcatheter arterial chemoembolization, systemic chemotherapy, and targeted therapy.

US 20180207253 provides a method of eliciting an immune response in a patient who has a cancer includes administering to said patient a composition containing a population of activated T cells that selectively recognize the cancer cells in the patient that aberrantly express a peptide consisting of the amino acid sequence of GVYDGEEHSV, in which the peptide is in a complex with an MHC molecule. US 20180258169 relates to use of anti-Claudin 1 monoclonal antibodies and pharmaceutical compositions thereof, for the prevention and/or treatment of hepatocellular carcinoma. US 20180162941 provides a method comprising determining the status of PD-1 on T cells, and based on a change in the level of PD-1 on certain cells, a determination of the effectiveness of the tyrosine kinase, and an indication for a combination therapy comprising a lower dose of tyrosine kinase inhibitor and a PD-1 inhibitor can be made.

However, these treatments exhibit limited survival benefit. HCC displays high recurrence rates after surgical treatments as well as high resistance to commonly used chemotherapeutic and targeted drugs, leading to poor patient survival.

### Summary of the Invention

Provided herein is a method for treating HCC comprising administering to the patient a combination of a dendritic cells-based vaccine and an immune checkpoint inhibitor. The combination is co-administered (or is for co-administration), e.g., according to a clinical dosage regimen disclosed herein (particular dose amounts given according to a specific dosing schedule).

In one embodiment, the dendritic cells-based vaccine is administered at a dose ranging from about 1×10⁵ cells/dose/day to about 1×10⁸ cells/dose/day. One embodiment includes administering the dendritic cells-based vaccine at a dose of about 1×10⁶ cells/dose/day. Certain embodiments of dendritic cells-based vaccine include but are not limited to the immature dendritic cell, mature dendritic cell, myeloid dendritic cells (cDCs), plasmacytoid dendritic cells (pDCs) and bone marrow-derived dendritic cell.

In one embodiment, the immune checkpoint inhibitor is administered at a dose ranging from about 50 µg/dose/day to about 400 µg/dose/day. One embodiment includes administering the immune checkpoint inhibitor at a dose about 100 µg/dose/day or about 200 µg/dose/day. Certain embodiments of the immune checkpoint inhibitor include antibodies directed against an immune checkpoint protein, such as an antibody directed against cytotoxic T-lymphocyte antigen 4 (CTLA-4 or CD152) or programmed cell death ligand-1 (PDL-1) or programmed cell death protein 1 (PD-1).

Dendritic cells-based vaccine and the immune checkpoint inhibitor are co-administered by infusion or injection. Dendritic cells-based vaccine and the immune checkpoint inhibitor may be provided as separate medicaments for administration at the same time or at different times. The co-administration is typically repeated on a cyclic basis, which may be repeated as appropriate over for instance 1 to 35 cycles. In one embodiment, an administration cycle comprising administration of dendritic cells-based vaccine and the immune checkpoint inhibitor every other day for three total doses. The co-administration may include simultaneous administration of the therapeutic agents (dendritic cells-based vaccine and an immune checkpoint inhibitor) in the same or different dosage form, or separate administration of the therapeutic agents.

Also provided is a medical kit for administering dendritic cells-based vaccine in combination with immune checkpoint inhibitor, comprising a printed instruction for administering dendritic cells-based vaccine and immune checkpoint inhibitor, and a combination of dendritic cells-based vaccine and immune checkpoint inhibitor in dosage units for at least one cycle.

### Brief Description of the Drawing

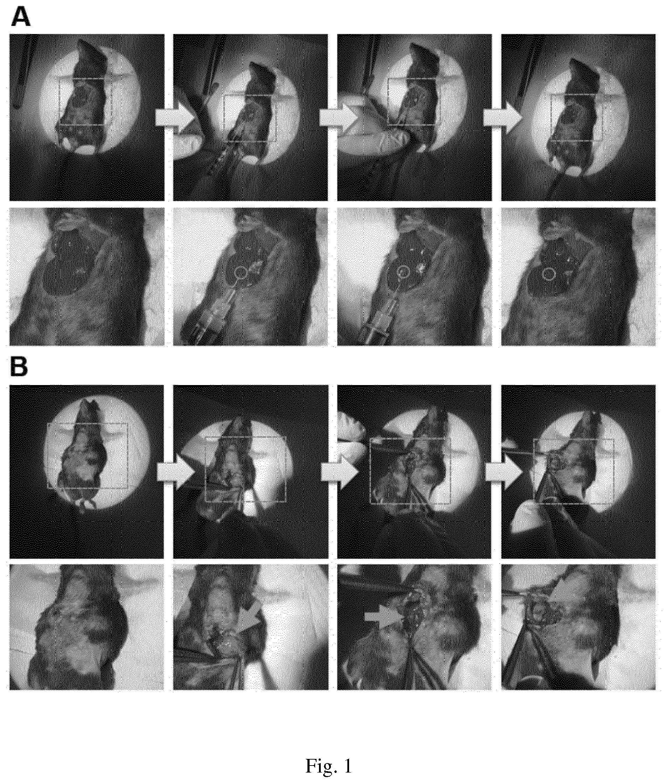

Figure 1 shows the establishment of the orthotopic HCC mouse model. (A) To establish an orthotopic HCC mouse model, the mouse hepatoma Hep-55.1C cells were directly injected into the left liver lobe of the mice undergoing midline laparotomy. The regions indicated with green dashed lines were magnified in the lower panel. The site of cell injection was indicated with green circles. (B) When mice died, tumors were observed to be developed orthotopically in the liver of mice following midline laparotomy. The regions indicated with green dashed lines were magnified in the lower panel. The orthotopic tumors were indicated with green arrows.

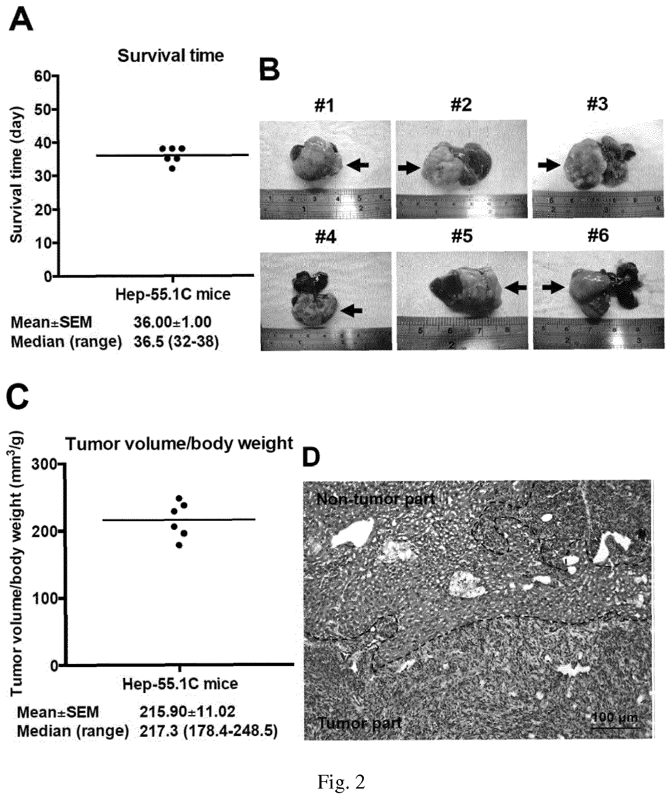

Figure 2 shows the validation of the orthotopic HCC mouse model. (A) Graphs showing the survival time and the ratios of liver weight, tumor weight, and tumor volume to body weight in the orthotopic HCC mice (n=6). The horizontal lines represented the mean values. The mean±SEM and median (range) values were shown below each graph. (B) Tumor growth in the liver of all six mice (denoted as #1 to #6). The tumors were indicated with black arrows. (C) Tumor histopathology by H&E staining. Black dashed lines defined the regions of tumor and non-tumor parts in the liver tissue. Scale bar was shown in the bottom right corner of the image. Original magnification, ×20.

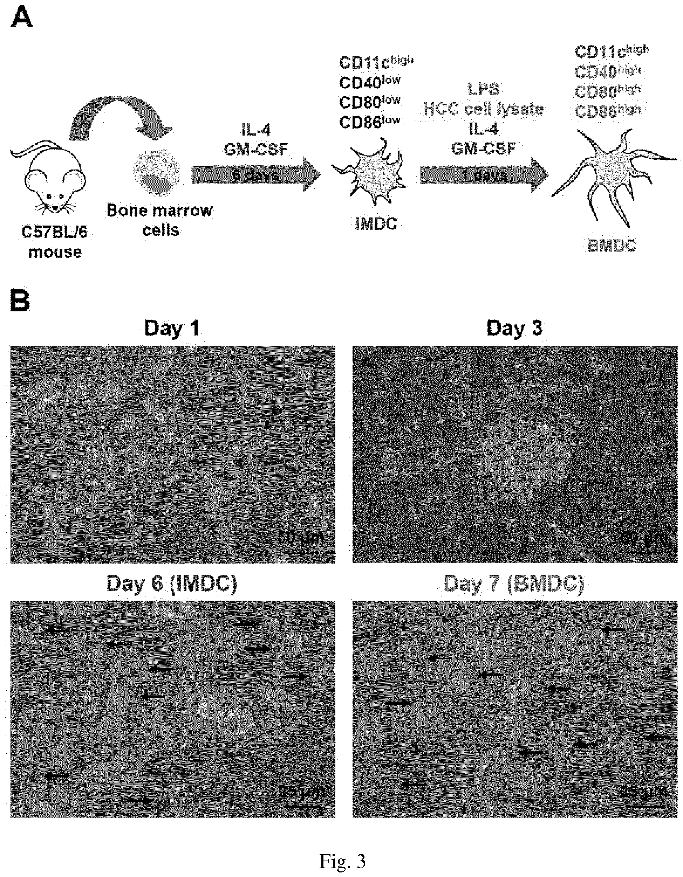

Figure 3 shows the generation and morphological characterization of the BMDC. (A) A schematic diagram illustrating the generation of the BMDC from mouse bone marrow. The IMDC expressed high levels of CD11c but low levels of CD40, CD80, and CD86 compared with the BMDC expressing high levels of these four molecules. (B) Cell morphology examined by inverted phase-contrast microscopy. Black arrows indicated the dendritic protrusions of the suspended cells. Scale bar was shown in the bottom right corner of each image. Original magnification, ×20 (Day 1 and Day 3); ×40 (Day 6 and Day 7).

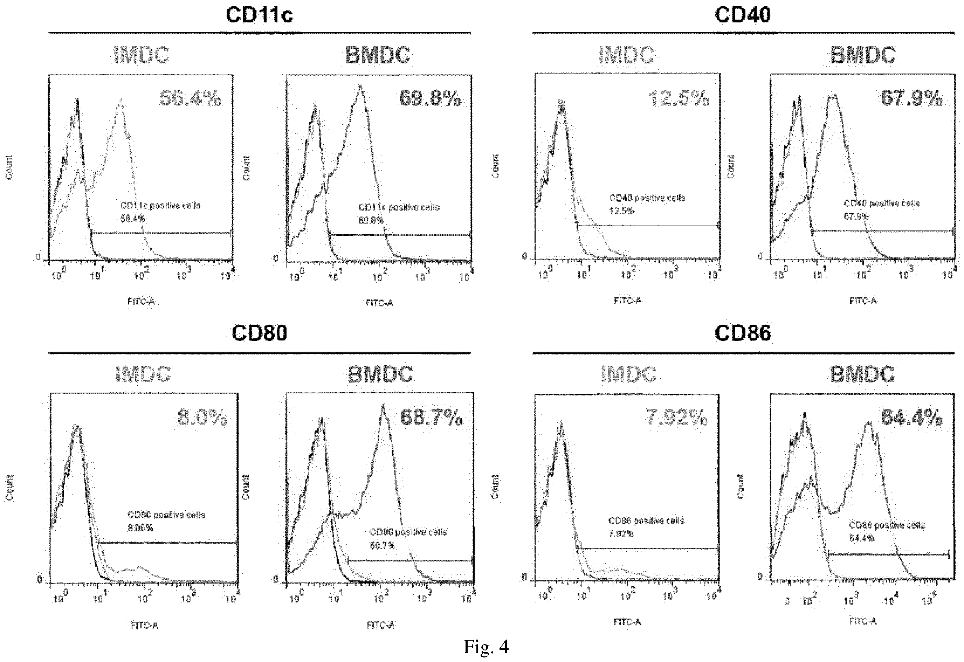

Figure 4 shows the phenotypical characterization of the BMDC. Flow cytometry analysis of the expression of the DC surface markers, including CD11c, CD40, CD80, and CD86 on the IMDC and BMDC. For the detection of each marker, the IMDC and BMDC were stained with antibodies of each marker (orange and red solid curves, respectively) or isotype-matched control antibodies (cyan solid curves) or remained unstained (black solid curves). The stained cells whose FITC intensity was higher than that of the cells stained with isotype-matched control antibodies were gated and considered as the cells positive for the indicated markers. The frequency of the cells expressing the indicated markers was calculated as the percentage of all analyzed cells and shown in the upper right corner of each graph.

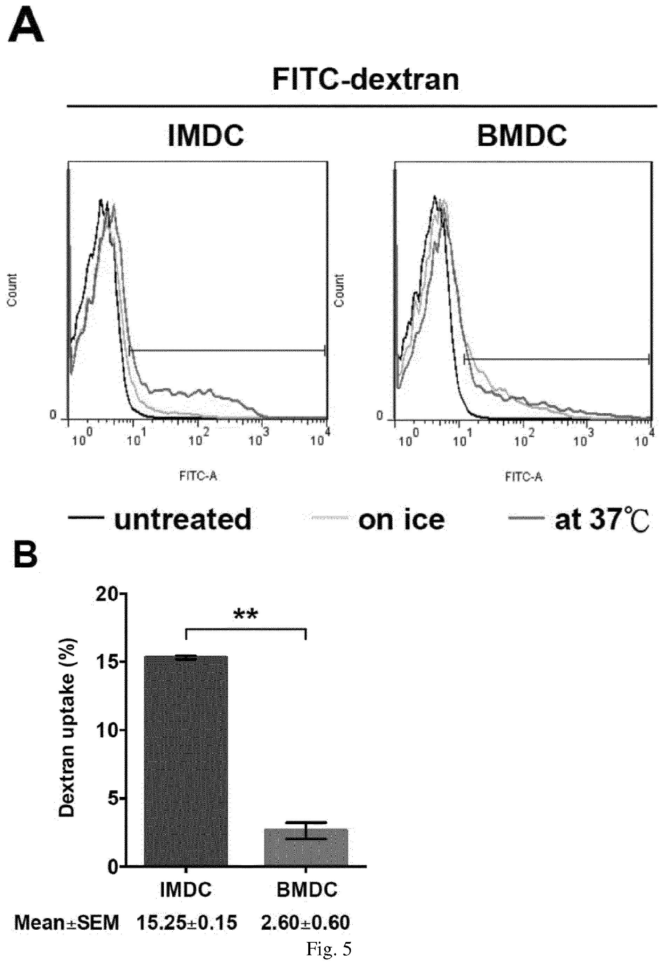

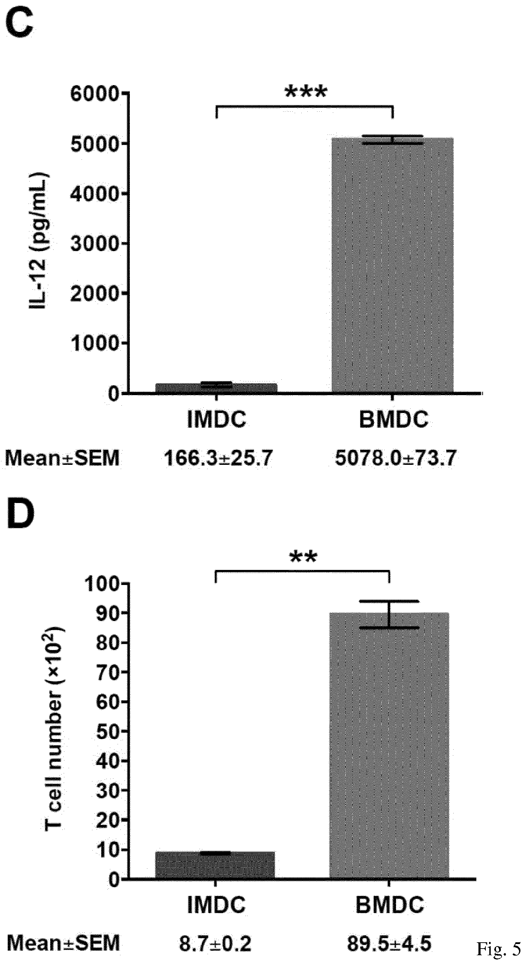

Figure 5 shows functional characterization of the BMDC. (A) For antigen uptake assay, the IMDC and BMDC were incubated with FITC-dextran at 37 °C (red solid curves) or on ice (cyan solid curves) or remained untreated (black solid curves), followed by flow cytometry analysis. The dextran-treated cells whose FITC intensity at 37 °C was higher than that on ice were gated and considered as the cells with the capacity to uptake dextran. Shown was the representative result of three independent experiments. (B) The frequency of the cells positive for FITC-dextran was calculated as the percentage of all analyzed cells. Data represented the mean with SEM error bar of three independent experiments. ***P* < 0.01. (C) IL-12 production by the IMDC and BMDC. The concentration of IL-12 in the culture supernatants of the IMDC and BMDC was measured by ELISA and expressed as the mean with SEM error bar of three independent experiments. ****P* < 0.001. (D) T cell proliferation induced by the IMDC and BMDC. After co-culture of T cells with the IMDC or BMDC in cell culture wells, the number of T cells in cell culture inserts was counted and shown as the mean with SEM error bar of three independent experiments. ***P* < 0.01.

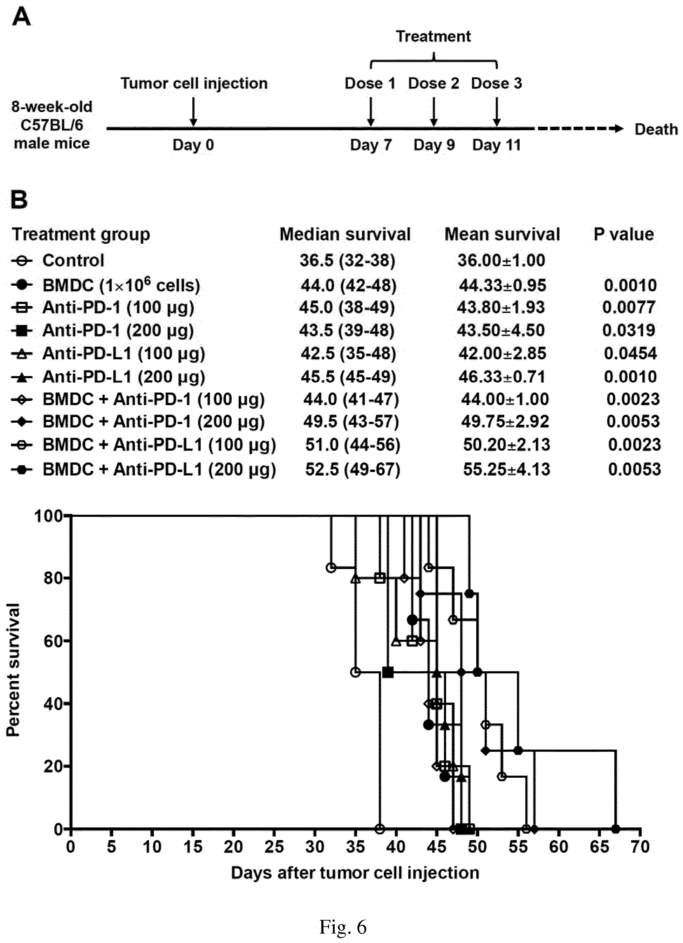

Figure 6 shows evaluation of the BMDC either alone or combined with PD-1/PD-L1 antibodies treatment in the orthotopic HCC mice. (A) Schematic timeline of the BMDC and/or anti-PD-1/PD-L1 treatment schedule in the orthotopic HCC mice. 8-week-old C57BL/6 male mice were injected with Hep-55.1C tumor cells on day 0. Treatment was started on day 7 after tumor cell injection and performed at 1-day intervals for a total of three doses. After treatment, all mice were followed until death to determine survival times. (B) Kaplan-Meier survival curves of the orthotopic HCC mice following treatment with the BMDC (1×10⁶ cells/dose) and/or anti-PD-1/PD-L1 (100 or 200 µg/dose). The cumulative survival rate was plotted against days after tumor cell injection. The overall survival in each group of mice (n=6) was shown as mean±SEM and median (range) in days. The significance of the difference of overall survival between different treatment groups of mice was analyzed and compared with the control group of mice. A *P* value < 0.05 was considered significant.

### Detailed Description of the Invention

Unless defined otherwise, all technical and scientific terms used herein have the same meaning as commonly understood by one of ordinary skill in the art to which the invention pertains. Generally, the nomenclature used herein and the experiment methods, which will be described below, are those well known and commonly employed in the art.

As used herein, the terms "a" and "an" and "the" and similar references used in the context can be construed to cover both the singular and the plural.

As used herein, the term "subject," "individual" or "patient" is used interchangeably herein, and refers to a vertebrate, preferably a mammal, more preferably a human.

As used herein, the terms "disease(s)", "disorder(s)", and "condition(s)" are used interchangeably, unless the context clearly dictates otherwise.

As used herein, a "combination" refers to any association between or among two or more items. The combination can be two or more separate items, such as two compositions or two collections, can be a mixture thereof, such as a single mixture of the two or more items, or any variation thereof. The elements of a combination are generally functionally associated or related.

As used herein, the term "treatment" or "treating" should be understood to include any indicia of success in the treatment, alleviation or amelioration of an injury, pathology or condition. This may include parameters such as abatement, remission, diminishing of symptoms, slowing in the rate of degeneration or decline, making the final point of degeneration less debilitating; improving a patient's physical or mental well-being; or, preventing the onset of disease.

As used herein, the term "therapeutically effective amount" when used in reference to symptoms of disease/condition refers to the amount and/or concentration of a compound that ameliorates, attenuates, or eliminates one or more symptom of a disease/condition or prevents or delays the onset of symptom(s).

As used herein, by "a combination" or "in combination with," it is not intended to imply that the therapy or the therapeutic agents must be administered at the same time and/or formulated for delivery together, although these methods of delivery are within the scope described herein. The therapeutic agents in the combination can be administered concurrently with, prior to, or subsequent to, one or more other additional therapies or therapeutic agents. The therapeutic agents or therapeutic protocol can be administered in any order.

Dendritic cells (DC) are the most potent antigen-presenting cells in the human immune system. In the immature state, DC are present in the blood and tissues, sampling antigens derived from virally infected, tumorigenic, or foreign cells. Upon uptake of presentable antigens, DC undergo maturation and antigen processing and migrate to lymph nodes, where they present antigens to and activate T cells and produce interleukin 12 (IL-12) to promote T cell proliferation, triggering the antigen-specific immune responses to destroy target cells. Based on these characteristics, DC-based immunotherapy, which stimulates tumor-specific immune responses, has emerged as a promising treatment strategy for HCC (Palucka K, Banchereau J. Cancer immunotherapy via dendritic cells. Nat Rev Cancer 2012;12: 265-277*;* Shang N, Figini M, Shangguan J, Wang B, Sun C, Pan L, Ma Q, Zhang Z. Dendritic cells based immunotherapy. Am J Cancer Res 2017;7: 2091-2102). Several clinical trials have been carried out to evaluate the efficacy of DC-based vaccine to treat HCC patients, for example, the DC pulsed with whole protein lysates of autologous human tumor cells or human hepatoma cell line HepG2 cells, as well as with peptides derived from known tumor antigens such as α-fetoprotein and glypican-3 (Butterfield LH, Ribas A, Potter DM, Economou JS. Spontaneous and vaccine induced AFP-specific T cell phenotypes in subjects with AFP-positive hepatocellular cancer. Cancer Immunol Immunother 2007;56: 1931-1943*;* Sawada Y, Yoshikawa T, Nobuoka D, Shirakawa H, Kuronuma T, Motomura Y, Mizuno S, Ishii H, Nakachi K, Konishi M, Nakagohri T, Takahashi S, Gotohda N, Takayama T, Yamao K, Uesaka K, Furuse J, Kinoshita T, Nakatsura T. Phase I trial of a glypican-3-derived peptide vaccine for advanced hepatocellular carcinoma: immunologic evidence and potential for improving overall survival. Clin Cancer Res 2012; 18: 3686-3696). Collectively, these clinical trials demonstrate that DC-based vaccine is safe and promising in the treatment of HCC patients. However, the overall results of current DC vaccination do not yet generate significant improvement in clinical outcomes. Therefore, new strategies are needed to increase the effectiveness of DC vaccine-induced immune responses to HCC.

In one embodiment, the present disclosure provides a method for treating HCC comprising co-administering to the patient, dendritic cells-based vaccine in combination with an immune checkpoint inhibitor. The treatment with DC vaccine in combination with immune checkpoint inhibitor (such as PD-1/PD-L1 antibodies) has significantly improved overall survival in subjects. Remarkably, combination treatment with DC vaccine and immune checkpoint inhibitor (such as PD-1/PD-L1 antibodies) led to longer overall survival of subjects than either treatment alone in a dose-dependent manner. DC vaccine combined with immune checkpoint inhibitor (such as PD-L1 antibodies) treatment exhibits better overall survival than that combined with immune checkpoint inhibitor (such as PD-1 antibodies). The present disclosure proves that combination therapy with DC vaccine and immune checkpoint inhibitor (such as PD-1/PD-L1 antibodies) may have great promise as a novel treatment strategy for HCC.

Dendritic cell (DC) vaccination in cancer patients aims to induce or augment an effective antitumor immune response against tumor antigens. In one embodiment, the dendritic cells-based vaccine is administered at a dose ranging from about 1×10⁵ cells/dose/day to about 1×10⁸ cells/dose/day. In one embodiment, the dendritic cells-based vaccine is administered at a dose of about 1×10⁶ cells/dose/day.

In one embodiment, examples of dendritic cells-based vaccine include but are not limited to the immature dendritic cell, mature dendritic cell, myeloid dendritic cells (cDCs), plasmacytoid dendritic cells (pDCs) and bone marrow-derived dendritic cell.

In the combination therapies provided herein, the immune checkpoint inhibitor can be an antibody directed against an immune checkpoint protein, such as an antibody directed against cytotoxic T-lymphocyte antigen 4 (CTLA-4 or CD152) or programmed cell death ligand-1 (PDL-1) or programmed cell death protein 1 (PD-1).

In one embodiment, the immune checkpoint inhibitor is administered at a dose ranging from about 50 µg/dose/day to about 400 µg/dose/day. In one embodiment, the immune checkpoint inhibitor is administered at a dose of about 100 µg/dose/day or about 200 µg/dose/day.

The anti-CTLA4 antibody refers to any antibody that specifically binds to cytotoxic T-lymphocyte-associated protein 4 (CTLA4) or a soluble fragment thereof and blocks the binding of ligands to CTLA4, thereby resulting in competitive inhibition of CTLA4 and inhibition of CTLA4-mediated inhibition of T cell activation. Hence, anti-CTLA4 antibodies are CTLA4 inhibitors. Reference to anti-CTLA4 antibodies herein include a full-length antibody and derivatives thereof, such as antigen-binding fragments thereof that specifically bind to CTLA4. Exemplary anti-CTLA4 antibodies include, but are not limited to, Ipilimumab or Tremelimumab, or a derivative or antigen-binding fragment thereof.

The anti-PD-1 antibody refers to any antibody that specifically binds to programmed cell death protein 1 (PD-1) or a soluble fragment thereof and blocks the binding of ligands to PD-1, thereby resulting in competitive inhibition of PD-1 and inhibition of PD-1-mediated inhibition of T cell activation. Hence, anti-PD-1 antibodies are PD-1 inhibitors. Reference to anti-PD-1 antibodies herein include a full-length antibody and derivatives thereof, such as antigen-binding fragments thereof that specifically bind to PD-1. Exemplary anti-PD-1 antibodies include, but are not limited to, Nivolumab, MK-3475, Pidilizumab, or a derivative or antigen-binding fragment thereof.

The anti-PD-Ll antibody refers to an antibody that specifically binds to programed death-ligand 1 (PD-L1) or a soluble fragment thereof and blocking the binding of the ligand to PD-1, thereby resulting in competitive inhibition of PD-1 and inhibition of PD-1-mediated inhibition of T cell activity. Hence, anti-PD-Ll antibodies are PD-1 inhibitors. Reference to anti-PD-Ll antibodies herein include a full-length antibody and derivatives thereof, such as antigen-binding fragments thereof that specifically bind to PD-L1. Exemplary anti-PD-L1 antibodies include, but are not limited to, BMS-936559, MPDL3280A, MEDI4736 or a derivative or antigen-binding fragment thereof.

In one embodiment, dendritic cells-based vaccine and the immune checkpoint inhibitor are administered in combination as part of an antitumor therapy. It is preferred to administer the combination by infusion or injection. Routes of administration by injection or infusion include intravenous, intraperitoneal, intramuscular, intrathecal and subcutaneous. Dendritic cells-based vaccine and the immune checkpoint inhibitor may be provided as separate medicaments for administration at the same time or at different times. In one embodiment, dendritic cells-based vaccine and the immune checkpoint inhibitor are provided as separate medicaments for administration at different times. When administered separately and at different times, it is preferable to administer dendritic cells-based vaccine followed by immune checkpoint inhibitor.

The medicaments suitable for administration to a patient are preferably in liquid form for infusion or injection. In general, medicaments typically comprise a pharmaceutically acceptable carrier. As used herein, the term "pharmaceutically acceptable" means approved by a government regulatory agency listed in the Pharmacopeia or another generally recognized pharmacopeia for use in animals, particularly in humans. The term "carrier" refers to a diluent, adjuvant, excipient, or vehicle with which the therapeutic agent is administered. Such pharmaceutical carriers can be sterile liquids, such as water and oils, including those of petroleum, animal, vegetable or synthetic origin, such as peanut oil, soybean oil, mineral oil, sesame oil and the like. Water or aqueous solution saline and aqueous dextrose and glycerol solutions may be employed as carriers, particularly for injectable solutions.

In one embodiment, the co-administration is typically repeated on a cyclic basis, which may be repeated as appropriate over for instance 1 to 35 cycles. In one embodiment, an administration cycle comprises administering dendritic cells-based vaccine and the immune checkpoint inhibitor every other day for three total doses.

The co-administration may include simultaneous administration of the therapeutic agents (dendritic cells-based vaccine and an immune checkpoint inhibitor) in the same or different dosage form, or separate administration of the therapeutic agents. For example, a dendritic cells-based vaccine may be simultaneously administered with the immune checkpoint inhibitor. Alternatively, a dendritic cells-based vaccine can be administered in combination with an immune checkpoint inhibitor, wherein both the dendritic cells-based vaccine and the immune checkpoint inhibitor are formulated for separate administration and are administered concurrently or sequentially. For example, the dendritic cells-based vaccine may be administered first, followed by the administration of the immune checkpoint inhibitor. Alternatively, the immune checkpoint inhibitor may be administered first, followed by administration of the dendritic cells-based vaccine.

In a further aspect of the present disclosure, a medical kit for administering dendritic cells-based vaccine in combination with immune checkpoint inhibitor is provided, comprising printed instructions for administering dendritic cells-based vaccine and immune checkpoint inhibitor according to the dosing schedules set forth above, and a combination of dendritic cells-based vaccine and immune checkpoint inhibitor in dosage units for at least one cycle, wherein each dosage unit contains the appropriate amount of the dendritic cells-based vaccine and immune checkpoint inhibitor for the treatments as defined above.

Co-administration can be carried out continuously or periodically within the maximum tolerated dose.

Although disclosure has been provided in some detail by way of illustration and example for the purposes of clarity of understanding, it will be apparent to those skilled in the art that various changes and modifications can be practiced without departing from the spirit or scope of the disclosure. Accordingly, the foregoing descriptions and examples should not be construed as limiting.

### Examples

### Material and methods

### Establishment of the orthotopic HCC mouse model

The orthotopic HCC mouse model was established as described.³¹ Briefly, 8-week-old immune-competent C57BL/6 male mice were anesthetized with isoflurane and subjected to midline laparotomy. 2×10⁶ of the mouse hepatoma Hep-55.1C cells, which were purchased from Cell Lines Service (Eppelheim, Germany) and maintained in DMEM medium (Invitrogen, Carlsbad, CA, USA) supplemented with 10% fetal bovine serum (FBS) (Gibco, Grand Island, NY, USA) and 1× penicillin/streptomycin (P/S) (Invitrogen), were directly injected into the left liver lobe of mice. Following hemostasis, the abdomen was closed in two layers. After surgery, the overall survival of mice was followed and the mice tumor burden was recorded when mice died. All animal experiments were performed under the approval of the institutional animal care and use committee of the China Medical University, Taichung, Taiwan.

### Measurement of tumor volume and histopathology

When mice died, the body weight of mice was recorded and liver was isolated for imaging. The tumor volume was calculated according to the equation V = 1/2 (L × W²), where V is the tumor volume, L the length, and W the width. To evaluate tumor histopathology, the formalin-fixed and paraffin-embedded liver tissues were sectioned into 4 µm thick for hematoxylin and eosin (H&E) staining.

### Generation of the HCC cell lysate-pulsed mature DC (BMDC)

In this study, the DC were derived from mouse bone marrow. First, bone marrow was obtained from femurs and tibias of 6-week-old C57BL/6 mice and was digested with collagenase, depleted of red blood cells, passed through a 100-µm filter, and then centrifuged to collect a cell pellet. Next, the cell pellet was resuspended and cultured at a density of 2×10⁵ cells/mL for 6 days in RPMI-1640 medium (Invitrogen) supplemented with 10% FBS (Gibco), 1× P/S (Invitrogen), 1× minimum essential medium non-essential amino acids (Invitrogen), 1 mM sodium pyruvate (Invitrogen), 100 ng/mL of human granulocyte macrophage colony-stimulating factor (GM-CSF) (Sino Biological Inc., Beijing, China), and 10 ng/mL of interleukin (IL)-4 (Sino Biological Inc.) at 37 °C in a humidified 5% CO₂ atmosphere. The culture medium and cytokines were refreshed on day 3 of culture. On day 6, the immature DC (IMDC) were harvested from the non-adherent and loosely adherent cells in the culture. To generate the BMDC, the IMDC were next cultured at a density of 1×10⁶ cells/mL in the above medium with the addition of 1 mg of freeze-thaw Hep-55.1C tumor cell lysate for 30 minutes, followed by the addition of 50 ng/mL of lipopolysaccharide (LPS) (Sigma, Louis, MO, USA) for another day. On day 7, all the cultured cells were collected as the BMDC and used as the DC vaccine.

### Flow cytometry analysis of DC phenotypes

The IMDC and BMDC were washed with phosphate-buffered saline (PBS), aliquoted into fractions (5×10⁵ cells/100 µL), and then stained for 30 minutes in the dark at room temperature with a final concentration of 5 µg/mL of the following antibodies purchased from BD Biosciences (San Jose, CA, USA): fluorescein isothiocyanate (FITC)-conjugated CD11c (553801), FITC-conjugated CD40 (553790), FITC-conjugated CD80 (553768), and FITC-conjugated CD86 (553691). As the negative or no-antibody control, the cells were also stained with corresponding FITC-conjugated isotype-matched control antibodies or remained unstained. After staining, the cells were washed with PBS twice and analyzed by a BD LSRII flow cytometer (BD Biosciences). Data analysis was performed using FlowJo software (Tree Star, San Carlos, CA, USA). The cells positive for FITC-CD11c were considered as DC that had successfully differentiated from bone marrow cells. The cells positive for FITC-CD40, FITC-CD80, and FITC-CD86 were considered as DC that had undergone successful maturation.

### FITC-conjugated dextran (FITC-dextran) uptake assay

The IMDC and BMDC were incubated with 1 mg/mL of FITC-dextran (MW4000; Sigma) at a density of 1×10⁶ cells/mL in RPMI-1640 medium (Invitrogen) supplemented with 10% FBS (Gibco) and 1× P/S (Invitrogen) for 30 minutes in the dark at 37 °C to allow for phagocytosis or on ice to stop phagocytosis as the negative control. After incubation, the cells were washed with ice-cold PBS twice and analyzed by a BD LSRII flow cytometer (BD Biosciences). Data analysis was carried out using FlowJo software (Tree Star). The FITC-positive cells were considered as cells that had successfully phagocytosed dextran. The experiments were performed three times independently.

### Detection of IL-12 production

The IMDC and BMDC were cultured at a density of 1×10⁶ cells/mL in RPMI-1640 medium (Invitrogen) supplemented with 10% FBS (Gibco) and 1× P/S (Invitrogen). After 1 day in culture, the supernatants were collected and measured by an enzyme-linked immunosorbent assay (ELISA) for IL-12 p70 using the Mouse IL-12 (p70) ELISA Set (BD Biosciences) following the manufacturer's instructions. The experiments were performed in triplicate three times independently.

### T cell proliferation assay

To isolate T cells, spleen cells were obtained from 6-week-old C57BL/6 mice using the same procedures described above as for bone marrow cells, followed by separation by a density gradient centrifugation with the Ficoll-Paque PLUS (density 1.077 g/mL; GE Healthcare, Uppsala, Sweden). The co-culture of DC and T cells was carried out by placing cell culture inserts (pore size 0.4 µm; Falcon, Oxnard, CA, USA) onto each well of a 24-well plate, followed by seeding DC and T cells into the wells and inserts at a density of 2×10⁵ cells/well and 1×10⁵ cells/insert, respectively, in RPMI-1640 medium (Invitrogen) supplemented with 10% FBS (Gibco) and 1× P/S (Invitrogen). After 3 days in culture, T cells were collected from each insert and were counted immediately by a Countess Automated Cell Counter (Invitrogen). The experiments were performed in triplicate three times independently.

### In vivo administration of the BMDC and PD-1/PD-L1 antibodies

The BMDC were generated as described above. The immune checkpoint inhibitors, the InVivoPlus anti-mouse PD-1 (BP0146) and PD-L1 (BP0101) monoclonal antibodies that have rigorous quality control measures, were purchased from Bio X Cell (West Lebanon, NH, USA). On day 7 after tumor cell injection, the orthotopic HCC mice were randomized into one of 10 treatment groups (6 mice/group): the vehicle control, the BMDC (1×10⁶ cells/dose), the anti-PD-1 (100 µg/dose), the anti-PD-1 (200 µg/dose), the anti-PD-L1 (100 µg/dose), the anti-PD-L1 (200 µg/dose), the BMDC (1×10⁶ cells/dose) plus anti-PD-1 (100 µg/dose), the BMDC (1×10⁶ cells/dose) plus anti-PD-1 (200 µg/dose), the BMDC (1×10⁶ cells/dose) plus anti-PD-L1 (100 µg/dose), and the BMDC (1×10⁶ cells/dose) plus anti-PD-L1 (200 µg/dose) treatment groups. BMDC were subcutaneously injected into the groin area (near lymph node) of mice. Anti-PD-1 and anti-PD-L1 antibodies were intraperitoneally injected into mice. Sterile PBS was used as the vehicle control and was injected into the control mice both subcutaneously and intraperitoneally, as well as the BMDC-treated mice and anti-PD-1/anti-PD-L1-treated mice intraperitoneally and subcutaneously, respectively. All treatments were begun on day 7 after tumor cell injection and repeated every other day for three total doses in each group of mice. After treatment, mice were followed until time of death to determine days of survival.

### Statistical analysis

The significance of the difference between IMDC and BMDC in their capacity to uptake dextran, produce IL-12, and stimulate T cell proliferation was determined by unpaired *t-*test. Data were represented as the mean with the standard error of the mean (SEM) error bar of three independent experiments. The significance of the difference of overall survival between different treatment groups of mice was determined by Kruskal-Wallis one-way ANOVA followed by Dunn's multiple comparisons test. A *P* value < 0.05 was considered significant (**P* < 0.05, ***P* < 0.01, ****P* < 0.001).

### Example 1 The orthotopic HCC mice develop tumors in liver and died about 32 to 38 days after inoculation of tumor cells

The orthotopic HCC mouse model was established as described in the Material and methods section (Figure 1). As shown in Figure 2A, the orthotopic HCC mice (Hep-55.1C mice, n=6) had mean and median survival times of 36 (SEM, 1.00) and 36.5 (range, 32 to 38) days, respectively, after inoculation of the tumor cells. When mice died, HCC tumors were observed to be orthotopically developed in the liver of all six mice (Figure 2B). The ratio (mean±SEM (median, range) of tumor volume to body weight was 215.90±11.02 mm³/g (217.3, 178.4 to 248.5) (Figure 2C). The tumor histopathology was evaluated by H&E staining (Figure 2D).

### Example 2 The BMDCs display appropriate morphology and phenotypes

The BMDCs were generated as described in the Material and methods section (Figure 3A). As shown in Figure 3B, compared with day 1 of culture, the cells increased gradually and began to form colonies in the suspension on day 3. On day 6 of culture, the cell volume apparently enlarged and the suspended cells began to form dendritic protrusions, a classical dendritic cell morphology, becoming the IMDC. Following incubation with Hep-55.1C tumor cell lysate and LPS for another day (day 7), the IMDC matured into the BMDC with the further elongated dendritic protrusions.

To evaluate the phenotypes of the BMDC, flow cytometry was performed to analyze the expression of the DC surface markers, including the identity marker CD11c and the maturation markers CD40, CD80, and CD86. As shown in Figure 4, the IMDC expressed high levels of CD11c but low levels of CD40, CD80, and CD86 compared with the BMDC expressing high levels of these four molecules. The data indicated that the BMDC we prepared had high purity and maturity.

### Example 3 The BMDCs exhibit optimal maturation with reduced uptake of antigen and increased capacity to produce IL-12 and promote T cell proliferation

The DC maturation process is associated with a loss of the capacity of DC to uptake antigens.¹² To compare the antigen uptake capacity between the IMDC and BMDC, the cells were incubated with FITC-dextran, followed by flow cytometry analysis. As expected, the BMDC exhibited significantly decreased levels of FITC-dextran uptake compared with the IMDC (mean±SEM, 2.60±0.60% versus 15.25±0.15%; *P* = 0.0023) (Figure 5A and 5B).

Mature DC can synthesize high levels of IL-12, which mediates the activation and proliferation of T cells during the engagement between DC and T cells.¹¹ Next, we assessed the capacities of the BMDC to secrete IL-12 and stimulate T cell proliferation. As shown in Figure 5C, IL-12 concentrations were significantly elevated in the culture supernatants of the BMDC compared with the IMDC (mean±SEM, 5078.0±73.7 pg/mL versus 166.3±25.7 pg/mL; *P <* 0.001). When co-cultured with T cells, the BMDC enhanced significantly higher levels of T cell proliferation than the IMDC (mean±SEM, 89.5±4.5×10² cells versus 8.7±0.2×10² cells; *P* = 0.0031) (Figure 5D). Collectively, these results indicated that the BMDC we prepared had optimal maturation and functions.

### Example 4 Combination treatment with the BMDCs and PD-1/PD-L1 antibodies leads to longer overall survival than either treatment alone in the orthotopic HCC mice

To evaluate the efficacy of BMDC combined with PD-1/PD-L1 antibodies for the treatment of HCC, the orthotopic HCC mice (6 mice/group) were administered with three total doses of the BMDC (1×10⁶ cells/dose) and/or PD-1/PD-L1 antibodies (100 or 200 µg/dose) at 1-day intervals and were followed for survival (Figure 6A). As shown in Figure 6B, the groups of mice treated with the BMDC or anti-PD-1/PD-L1 had significantly improved overall survival (days, mean±SEM (median, range) than the control group of mice (BMDC (1×10⁶ cells/dose): 44.33±0.95 (44.0, 42 to 48); anti-PD-1 (100 µg/dose): 43.80±1.93 (45.0, 38 to 49); anti-PD-1 (200 µg/dose): 43.50±4.50 (43.5, 38 to 48); anti-PD-L1 (100 µg/dose): 42.00±2.85 (42.5, 35 to 48); anti-PD-L1 (200 µg/dose): 45.80±0.58 (45.0, 45 to 48); control: 36.00±1.00 (36.5, 32 to 38). At 38 days after treatment, all mice in the control group had died, whereas almost all of mice treated with the BMDC or anti-PD-1/PD-L1 were still alive and had the longest survival times of about 48 to 49 days. However, no apparent dose-dependent effects on overall survival of mice were observed for the anti-PD-1/PD-L1 treatment.

Remarkably, combination treatment with the BMDC and anti-PD-1/PD-L1 could further prolong the overall survival of mice compared with either treatment alone (BMDC + anti-PD-1 (200 µg/dose): 49.75±2.92 (49.5, 43 to 57); BMDC + anti-PD-L1 (100 µg/dose): 50.20±2.13 (51.0, 44 to 56); BMDC + anti-PD-L1 (200 µg/dose): 55.25±4.13 (52.5, 49 to 67), except for the BMDC + anti-PD-1 (100 µg/dose) treatment to a lesser extent (44.00±1.00 (44.0, 41 to 47) (Figure 6B). The longest survival times of mice were extended from 48 days by the BMDC (1×10⁶ cells/dose), anti-PD-1 (200 µg/dose), or anti-PD-L1 (100 or 200 µg/dose) single treatment to 57 days by the BMDC + anti-PD-1 (200 µg/dose), to 56 days by the BMDC + anti-PD-L1 (100 µg/dose), and to 67 days by the BMDC + anti-PD-L1 (200 µg/dose) combined treatment. Moreover, the BMDC combined with anti-PD-1/PD-L1 treatment prolonged the overall survival of mice in a dose-dependent manner. The groups of mice treated with the BMDC and anti-PD-L1 exhibited better overall survival than those treated with the BMDC and anti-PD-1.

### Example 5 Clinical Trial Study

A clinical trial study was made to assess efficacy of the combination treatment with the BMDC and anti-PD-1/PD-L1. In the combination therapy, BMDCs at a dose ranging from about 1×10⁵ cells/dose/day to about 1×10⁸ cells/dose/day were administrated to HCC patients, followed by treatment of anti-PD-L1 or anti-PD-1 at a dose ranging from about 50 µg/dose/day to about 400 µg/dose/day. On firsst day, the subject will receive the primer vaccine dose; this will be followed by two booster vaccine doses at 6 weeks apart. Peripheral blood will be taken weekly to monitor the immune response to each peptide by tetramer assay. Anti PD-1 therapy was commence 5-8 weeks after the subject's last dendritic cell vaccine. The preliminary evaluation shows that the patients receiving the combination therapy exhibits singificantly tumor shrinkage.

### Additional Aspects of the Invention

The present invention also provides the following.
1. A combination of a dendritic cells-based vaccine and an immune checkpoint inhibitor for use in the treatment of a hepatocellular carcinoma (HCC) by co-administration.
2. The combination according to 1, wherein the dendritic cells-based vaccine is administered at a dose ranging from about 1×105 cells/dose/day to about 1×108 cells/dose/day.
3. The combination according to 1 or 2, wherein the dendritic cells-based vaccine is administered at a dose of about 1×106 cells/dose/day.
4. The combination according to any one of 1 to 3, wherein the dendritic cells-based vaccine is immature dendritic cell, mature dendritic cell, myeloid dendritic cells (cDCs), plasmacytoid dendritic cells (pDCs) or bone marrow-derived dendritic cell.
5. The combination according to any one of 1 to 4, wherein the immune checkpoint inhibitor is an antibody directed against cytotoxic T-lymphocyte antigen 4 (CTLA-4 or CD152), programmed cell death ligand-1 (PDL-1) or programmed cell death protein 1 (PD-1).
6. The combination according to any one of 1 to 5, wherein the immune checkpoint inhibitor is administered at a dose ranging from about 50 µg/dose/day to about 400 µg/dose/day.
7. The combination according to any one of 1 to 6, wherein the immune checkpoint inhibitor is administered at a dose of about 100 µg/dose/day or about 200 µg/dose/day.
8. The combination according to any one of 1 to 7, wherein the dendritic cells-based vaccine in combination with an immune checkpoint inhibitor is administered by infusion or injection.
9. The combination according to any one of 1 to 8, co-administration is through the route of intravenous, intraperitoneal, intramuscular, intrathecal or subcutaneous.
10. The combination according to any one of 1 to 9, wherein the dendritic cells-based vaccine and the immune checkpoint inhibitor may be provided as separate medicaments for administration at the same time or at different times.
11. The combination according to any one of 1 to 10, wherein the co-administration is repeated on a cyclic basis.
12. The combination according to any one of 1 to 11, wherein an administration cycle comprises administering the dendritic cells-based vaccine and the immune checkpoint inhibitor every other day for three total doses.
13. The combination according to any one of 1 to 12, wherein the co-administration comprises simultaneous administration or separate administration of the dendritic cells-based vaccine and the immune checkpoint inhibitor.
14. The combination according to any one of 1 to 13, wherein the dendritic cells-based vaccine and the immune checkpoint inhibitor are formulated for separate administration and are administered concurrently or sequentially.
15. The combination according to any one of 1 to 14, wherein the dendritic cells-based vaccine may be administered first, followed by the administration of the immune checkpoint inhibitor.

## Claims

1. A method for treating a hepatocellular carcinoma (HCC) comprising co-administering to the patient, a dendritic cells-based vaccine in combination with an immune checkpoint inhibitor.

2. The method of Claim 1, wherein the dendritic cells-based vaccine is administered at a dose ranging from about 1×10⁵ cells/dose/day to about 1×10⁸ cells/dose/day.

3. The method of Claim 1, wherein the dendritic cells-based vaccine is administered at a dose of about 1×10⁶ cells/dose/day.

4. The method of Claim 1, wherein the dendritic cells-based vaccine is immature dendritic cell, mature dendritic cell, myeloid dendritic cells (cDCs), plasmacytoid dendritic cells (pDCs) or bone marrow-derived dendritic cell.

5. The method of Claim 1, wherein the immune checkpoint inhibitor is an antibody directed against cytotoxic T-lymphocyte antigen 4 (CTLA-4 or CD152) or programmed cell death ligand-1 (PDL-1) or programmed cell death protein 1 (PD-1).

6. The method of Claim 1, wherein the immune checkpoint inhibitor is administered at a dose ranging from about 50 µg/dose/day to about 400 µg/dose/day.

7. The method of Claim 1, wherein the immune checkpoint inhibitor is administered at a dose of about 100 µg/dose/day or about 200 µg/dose/day.

8. The method of Claim 1, wherein the dendritic cells-based vaccine in combination with an immune checkpoint inhibitor is administered by infusion or injection.

9. The method of Claim 1, co-administration is through the route of intravenous, intraperitoneal, intramuscular, intrathecal or subcutaneous.

10. The method of Claim 1, wherein the dendritic cells-based vaccine and the immune checkpoint inhibitor may be provided as separate medicaments for administration at the same time or at different times.

11. The method of Claim 1, wherein the co-administration is repeated on a cyclic basis.

12. The method of Claim 11, wherein an administration cycle comprises administering the dendritic cells-based vaccine and the immune checkpoint inhibitor every other day for three total doses.

13. The method of Claim 11, wherein the co-administration comprises simultaneous administration or separate administration of the dendritic cells-based vaccine and the immune checkpoint inhibitor.

14. The method of Claim 13, wherein the dendritic cells-based vaccine and the immune checkpoint inhibitor are formulated for separate administration and are administered concurrently or sequentially.

15. The method of Claim 14, wherein the dendritic cells-based vaccine may be administered first, followed by the administration of the immune checkpoint inhibitor.

16. A medical kit for administering dendritic cells-based vaccine in combination with immune checkpoint inhibitor, comprising a printed instruction for administering dendritic cells-based vaccine and immune checkpoint inhibitor, and a combination of dendritic cells-based vaccine and immune checkpoint inhibitor in dosage units for at least one cycle.
